# EUROPEAN PATENT SPECIFICATION

(11) **EP 3 596 443 B1**
(45) Date of publication and mention of the grant of the patent: **01.01.2025**
(21) Application number: 18767035.1
(22) Date of filing: 13.03.2018
(51) Int. Cl.: G01N 1/22, G01N 1/24, G01N 1/26, G01N 21/11, G01N 21/25, G01N 33/28, G01N 21/3504, G01N 21/03, G01N 21/33, G01N 21/65, G01N 21/17, G01N 21/35, G01N 21/39, H01F 27/12

(54) **DISSOLVED GAS ANALYSIS DEVICES, SYSTEMS, AND METHODS**
VORRICHTUNGEN, SYSTEME UND VERFAHREN ZUR ANALYSE VON GELÖSTEM GAS
DISPOSITIFS, SYSTÈMES ET PROCÉDÉS D'ANALYSE DES GAZ DISSOUS

(30) Priority: 13.03.2017 US 201715458010; 13.03.2017 US 201715458014
(43) Date of publication of application: 22.01.2020
(73) Proprietor: ABB Schweiz AG, 5400 Baden (CH)
(72) Inventor: BUIJS, Henry L., Quebec City, Québec G1T 2A6 (CA); BIBEAU, Louis-Philippe A., Saint-Augustin-De-Desmaures, Québec G3A1C2 (CA); OUELLET-BERLANGER, Alex, Boischatel, Québec G0A 1H0 (CA); DESBIENS, Raphael N., Québec City, Québec G1C 8C2 (CA)
(74) Representative: Aipex B.V.
(86) International application number: PCT/CA2018/050298
(87) International publication number: WO 2018/165749

(56) References cited:
- EP-A1- 2 746 747
- WO-A1-2013/116799
- WO-A1-98/20324
- JP-A- S57 156 536
- US-A1- 2008 088 821
- US-A1- 2016 266 085
- US-A1- 2016 266 085
- US-B1- 6 526 805
- US-B2- 8 014 965

## Description

### TECHNICAL FIELD

The present disclosure relates to the field of dissolved gas analysis. More particularly, the present disclosure relates to dissolved gas analysis for transformers.

### BACKGROUND

Electromagnetic devices, such as electrical transformers, can experience electrical inefficiencies and can generate significant heat in operation. Abating electrical inefficiencies and removing excess heat from such devices can conserve operational life, performance, and reduce the maintenance needs of the devices. Fluids, such as dielectric fluids, can be used as a cooling medium to remove heat from the devices and can provide an electrical insulation layer to suppress corona and arcing.

In operation, such cooling and/or insulating fluids can develop dissolved gases. Analysis of dissolved gases within the fluids of transformers can reveal useful information regarding the status of transformer operation.

US 2016/0266085, on which the preamble of claim 1 is based, discloses an apparatus for detecting gas in a high-voltage device filled with an insulating medium. The apparatus comprises an inlet for introducing a carrier gas and an outlet for discharging a carrier gas; at least one gas sensor for detecting a gas; a first pump for conveying the carrier gas in the apparatus; a membrane which at least consists of at least one semipermeable material, is at least partially surrounded by the insulating medium and is at least partially subjected to a flow of the carrier gas; a second pump for conveying the carrier gas into the apparatus and for conveying the carrier gas out of the apparatus; wherein there is no valve which can be used to convey the carrier gas into or out of the apparatus.

US 2008/0088821 discloses an apparatus in which densities of water vapor can be detected and quantified at a high sampling rate for a gas. The gas can be contained within a sample chamber within or outside of an instrument enclosure. A first split beam passes through the enclosure and the sample chamber while a second split beam that passes only through the enclosure provides a reference that can be used to correct for ambient humidity in the instrument enclosure.

EP 2746747 discloses a sensor assembly for sensing a hydrogen and moisture content of insulation liquid of a liquid-filled electrical equipment based on radiation detection. The sensor assembly comprises an electromagnetic radiation source for emitting electromagnetic radiation; a water detection section arranged for receiving a water-containing component of the insulation liquid when the sensor assembly is in operational connection with the electrical equipment and for being illuminated by electromagnetic radiation from the electromagnetic radiation source; a first electromagnetic radiation detector configured for detecting electromagnetic radiation coming from the water detection section at a wavelength indicative of an amount of water present at the water detection section; a hydrogen detection section arranged for receiving at least a hydrogencontaining component of the insulation liquid when the sensor assembly is in operational connection with the electrical equipment and for being illuminated by electromagnetic radiation from the at least one electromagnetic radiation source; and a second electromagnetic radiation detector configured for detecting electromagnetic radiation coming from the hydrogen detection section at a wavelength indicative of an amount of hydrogen present at the hydrogen detection section.

### SUMMARY

According to an aspect of the invention, there is provided a transformer according to claim 1.

The gas cell is arranged for determining characteristics of gas extracted from the fluid. In some embodiments, a transport conduit may be fluidly coupled with each of the extraction coil and the gas cell to transport gas received from the fluid to the gas cell for analysis. In some embodiments, the extraction coil may be formed as a conduit having an inner volume for receiving gas permeating through the gas-permeable material. In some embodiments, a gas species that is both within the inner volume and dissolved in the fluid may be in equilibrium.

In some embodiments, the gas-permeable material may include a fluoropolymer. In some embodiments, the gas-permeable material may include a fluoroplastic having at least one of: a yield strength within the range of about 26 MPa to about 29 MPa at about 73 °F, a yield strength within the range of about 0.5 MPa to about 13 MPa at about 302 °F, a yield strength within the range of about 4 MPa to about 13 MPa at about 428 °F, a tensile strength within the range of about 24 MPa to about 29 MPa at about 73 °F, a tensile strength within the range of about 1 MPa to about 15 MPa at about 302 °F, and a tensile strength within the range of about 3 MPa to about 7 MPa at about 428 °F. In some embodiments, the fluoropolymer may include a fluoroplastic having optical transmission percent of greater than 95. In some embodiments, the fluoroplastic may include a fluoroplastic having gas permeability of at least one of H₂O of about 1142 Barrer, O₂ of about 340 Barrer, and N₂ of about 130 Barrer.

### BRIEF DESCRIPTION OF THE DRAWINGS

The concepts described in the present disclosure are illustrated by way of example and not by way of limitation in the accompanying figures. For simplicity and clarity of illustration, elements illustrated in the figures are not necessarily drawn to scale. For example, the dimensions of some elements may be exaggerated relative to other elements for clarity. Further, where considered appropriate, reference labels have been repeated among the figures to indicate corresponding or analogous elements. The detailed description particularly refers to the accompanying figures in which:
Figure 1 is a diagrammatic view of an electrical transformer showing that the transformer includes a gas analysis system for determining characteristics of dissolved gases within fluid of the transformer, and that the gas analysis system includes a gas-permeable extraction probe for extracting gases from the fluid and a gas analysis module for performing analysis on the extracted gas, and showing that the extraction probe and the analysis module are fluidly connected to form a gas circuit;
Figure 2 is an elevation view of an illustrative embodiment of a sample portal of the electrical transformer of Figure 1 in partial cross-section to show that an extraction module including the extraction probe is arranged to place the extraction probe in contact with the fluid of the transformer;
Figure 3 is a perspective view of an illustrative embodiment of the extraction module showing that the extraction probe is mounted on a frame to form the extraction module;
Figure 4 is another perspective view of the extraction module of Figure 3 showing that the extraction probe is formed as an extraction coil and is mounted on a spool of the frame;
Figure 5 is a diagrammatic view of the gas analysis module showing that the gas analysis module includes a gas cell containing extracted gases and a reference space containing ambient gases, and showing that the gas analysis module includes a light source providing a first channel for measuring gas within the gas cell by passing a first light beam through the gas cell for reception by a first detector, and a second channel for measuring gas within the reference space by passing a second light beam through the reference space for reception by a second detector;
Figure 6 is a perspective view of the gas cell of the gas analysis module showing that the gas cell includes windows for passing light; and
Figure 7 is a flow diagram illustrating a process of the gas analysis system for determining characteristics of dissolved gases within fluid of the transformer.

### DETAILED DESCRIPTION OF THE DRAWINGS

While the concepts of the present disclosure are susceptible to various modifications and alternative forms, specific embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives consistent with the present disclosure and the appended claims.

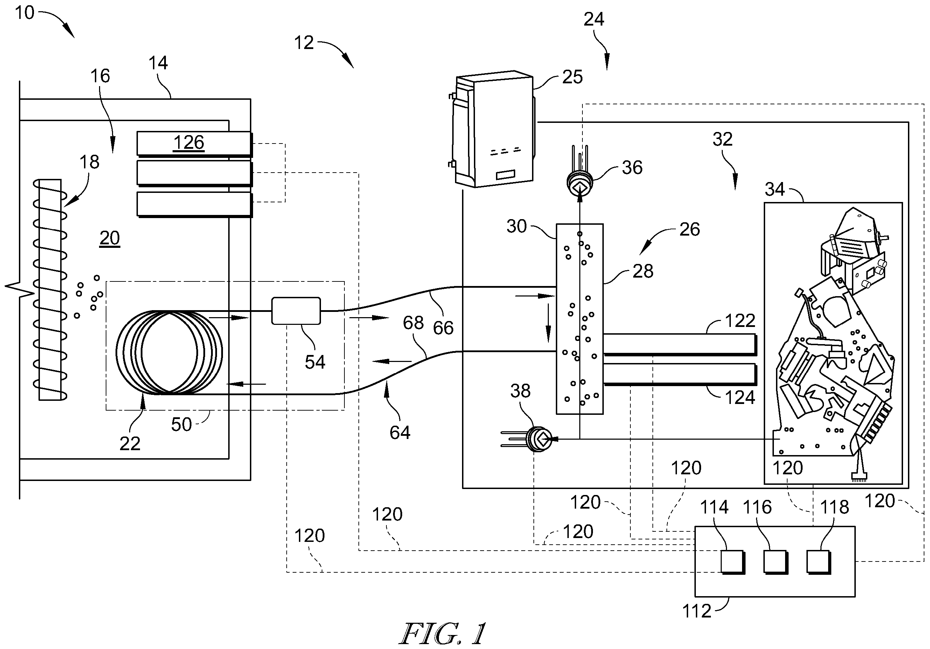

Figure 1 shows an illustrative arrangement of an electrical transformer 10 including a gas analysis system 12 for determining characteristics of dissolved gases within fluid of the transformer 10. The transformer 10 illustratively includes a housing 14 defining an interior 16 and electrical windings 18 arranged within the interior 16 of the housing 14. The electrical windings 18 illustratively comprise windings of electrical wiring forming a series of turns about limbs of the transformer 10 to produce electromagnetic effect when current is passed through the wiring. The transformer 10 is illustratively embodied as a high- voltage, three-phase, core type transformer, but in some embodiments, may include any manner of electromagnetic device including but not limited to shell type and/or single or multi-phase.

In the illustrative embodiment as shown in Figure 1, the housing 14 contains fluid 20 for cooling and/or electrically insulating the components of the transformer 10, such as the electrical windings 18. As mentioned above, dissolved gases can develop within the fluid 20 as a result of operable use of the fluid 20 for cooling and/or insulation (for example, by fluid breakdown and/or faulty operational issues of the transformer 10, generally, including leaks in the housing 14) and/or the fluid 20 may carry gases generated from the degradation of other insulation materials in the transformer, such as paper. The gas analysis system 12 is illustratively arranged to extract dissolved gases from the fluid 20 for analysis.

Referring to Figure 1, the gas analysis system 12 includes an extraction probe 22 for extracting gas from the fluid 20. The extraction probe 22 is arranged in contact with the fluid 20 and is formed of a gas-permeable material to permit permeation of dissolved gases from the fluid 20. The extraction probe 22 is illustratively embodied as a conduit defining an interior passage for receiving and communicating gas. The gas-permeable material illustratively permits dissolved gases to permeate into the interior passage while inhibiting ingress of liquids, for example, dielectric oils. Suitable gas-permeable materials may include one or more fluoropolymers. The extraction probe 22 is formed as an extraction coil having coil loops in contact with the fluid 20. In other examples not forming part of the claimed invention, the extraction probe 22 may include any suitable shapes and/or forms.

As shown in Figure 1, the gas analysis system 12 includes a gas analysis module 24 for conducting analysis of gas. The gas analysis module 24 is fluidly connected with the extraction probe 22 and forms a gas circuit for circulation of gas between the extraction probe 22 and the gas analysis module 24. An exemplary housing 25 of the gas analysis module 24 is shown in Figure 1. The gas analysis module 24 illustratively includes a gas cell 26 for receiving gas extracted from the fluid 20. The gas cell 26 illustratively includes a cell body 28 defining a cavity 30 through which gas is passed for analysis. In the illustrative embodiment, the gas analysis system 12 conducts optical analysis of gas to determine gas characteristics. In the illustrative embodiment, portions of the gas circuit other than the extraction probe 22, including the cavity 30, are hermetically sealed to ambient air such that only the extraction probe 22 is arranged to allow permeation of gases into and out of the gas circuit, thereby allowing gas exchange with the transformer fluid 20.

As shown in Figure 1, the gas analysis module 24 illustratively includes a gas analysis device 32 for conducting analysis of gas within the gas cell 26. In the illustrative embodiment, the gas analysis device 32 is an optical device embodied as a light spectroscopy device, namely a Fourier transform infrared (FTIR) spectrometer. In some embodiments, the gas analysis module 24 may perform any manner of gas analysis techniques and may include any suitable configuration and/or components to perform such techniques, for example, but without limitation, ultra violet light spectroscopy, Raman spectroscopy, photoacoustic spectroscopy, tunable diode laser absorption spectroscopy (TDLAS).

The gas analysis device 32 illustratively performs light spectrum analysis of gas within the gas cell 26. In some embodiments, the gas cell 26 may use optical path length enhancement techniques such as multi-pass cells or resonant cavities. Multi-pass cells may include White cell, Herriot cell, folded path cells, and/or other multi-pass cells. Resonant cavities may include Fabry-Perot cavities, cavities designed for cavity ring-down spectroscopy, integrated cavity output spectroscopy (ICOS), off-axis integrated cavity output spectroscopy (OA-ICOS), and/or other optical path length enhancement techniques.

As shown in Figure 1, the gas analysis device 32 illustratively includes a light source 34 and detectors 36, 38 for receiving light from the light source 34. As discussed in additional detail below, the light source 34 illustratively generates infrared (IR) light for propagation through gas for observation of the light absorption characteristics of the gas. The light directed through the gas is received by the detectors 36, 38. The detectors 36, 38 are illustratively embodied as photodetectors that receive light propagated through gas (but that has not been absorbed by the gas) and that generate an electrical signal indicating the light received. The detectors 36, 38 are illustratively embodied as analog detectors that generate an analog signal that is converted to a digital signal by an analog-to-digital converter. In some embodiments, the detectors 36, 38 may include any suitable arrangement of signal generation for gas analysis.

The gas analysis device 32 illustratively determines characteristics of the gas based on the light received by the detectors 36, 38. In the illustrative embodiments, the gas analysis module 24 can determine characteristics of dissolved gas within the fluid 20 by analysis of gas extracted by the gas analysis system 12 from the transformer 10. Relevant characteristics of dissolved gases within the fluid 20 of the transformer 10 include the presence and/or identification of such gases and their dissolved concentrations within the fluid 20. A non- exhaustive list of gases of interest within the fluid 20 may include, for example, oxygen (O₂), nitrogen (N₂), hydrogen (H₂), carbon dioxide (CO₂), and/or hydrocarbons (e.g., methane, ethane, acetylene, and/or ethylene), among other gases. The gas analysis device 32 may also monitor water vapor (H₂O) extracted from the moisture dissolved in transformer oil 20.

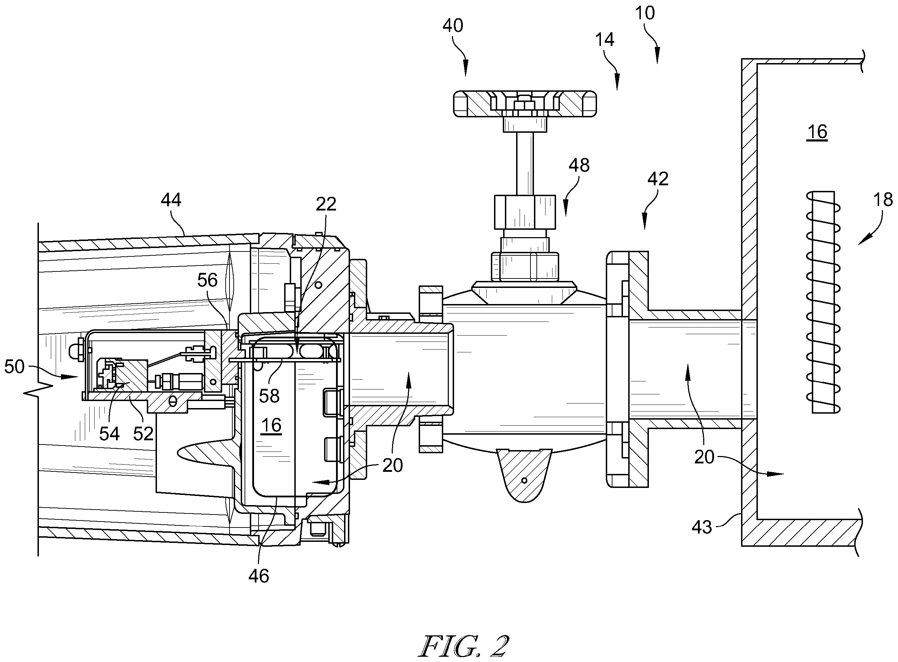

Referring now to Figure 2, the transformer 10 is shown in partial cross-section for descriptive purposes. The housing 14 of the transformer 10 illustratively includes a sampling portal 40 defining a portion of the interior 16 containing fluid 20 as part of the housing 14. The sampling portal 40 illustratively includes pipe extension 42 connected with a wall 43 of the transformer 10 and a shroud 44 secured with the pipe extension 42. The extraction probe 22 is illustratively mounted within the shroud 44 in contact with fluid 20. The extraction probe 22 is illustratively mounted within a fluid chamber 46 defined by the shroud 44 as a part of the interior 16. The chamber 46 illustratively contains fluid 20 as part of the housing 14 and fluidly communicating through the pipe extension 42. In the illustrative embodiment, the pipe extension 42 illustratively includes a valve 48 disposed fluidly between the wall 43 and the chamber 46 to permit isolation of the extraction probe 22, but in some embodiments, the valve 48 may be excluded. In some embodiments, the extraction probe 22 may be arranged inside of the wall 43.

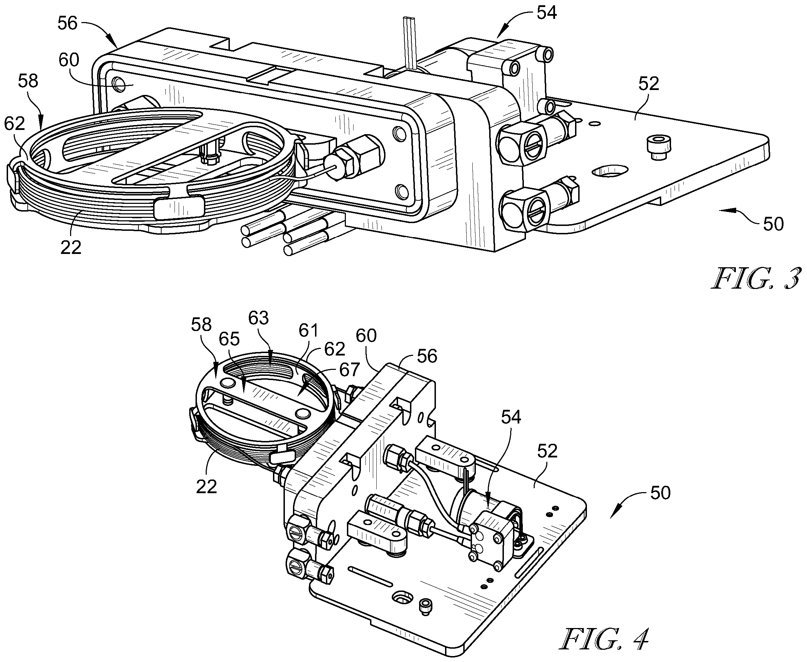

The gas analysis system 12 includes an extraction module 50 as shown in Figures 2-4, The extraction module 50 provides a packaging platform for mounting the extraction probe 22 within the housing 14 as shown in Figure 2. Referring to Figures 3 and 4, the extraction module 50 includes a mounting frame 52 and the extraction probe 22 secured with the mounting frame 52. In the illustrative embodiment, a pump 54 is mounted to the frame 54 and is fluidly connected with the extraction probe 22 to provide a motive pressure source for circulation of gas within the gas circuit. Control valves and/or other flow distribution devices for operation of the gas circuit may be mounted to the mounting frame 52.

As shown in Figures 3 and 4, the mounting frame 52 includes an engagement wall 56 and a probe arm 58 extending from the engagement wall 56. The engagement wall 56 illustratively includes a face 60 that forms at least a portion of fluid boundary of the chamber 46. The engagement wall 56 illustratively supports the probe arm 58 for extension within the chamber 46 for contact with fluid 20.

In the illustrative embodiment as shown in Figures 3 and 4, and according to the invention, the probe arm 58 includes a spool 62 having the extraction probe 22 (which according to the invention is an extraction coil) looped around the spool 62. In the illustrative embodiment, the extraction coil is looped around the spool 62 to form a number of coil turns having a successively stacked arrangement for exposure to fluid 20. Increasing the number of coils may improve the effective exchange surface between oil and gas phase and may reduce the response time of the measurement. Gas circulated through the extraction probe 22 portion of the gas circuit illustratively flows successively through each of the coil turns and out for circulation to the gas analysis device 32. In the illustrative embodiment, the extraction probe 22 is fluidly connected with the pump 54 for communication of extracted gas through the gas circuit.

As best shown in Figure 4, the spool 62 is illustratively cantilevered from the engagement wall 56 and provides structure for arranging the extraction probe 22 for contact with fluid 20. In some embodiments, the extraction probe 22 may be secured to the mounting frame 52 in any suitable manner and/or arrangement. The spool 62 is illustratively formed as a structural frame defining an annular spool bed 61 for receiving the extraction probe 22 wrapped thereon and defining openings 63 extending through the spool bed 61 to permit fluid 20 to contact interior portions of the extraction probe 22 to increase the effective exchange surface between oil and gas phase. The spool 62 is illustratively shaped as a hollow cylinder to permit fluid 20 therein. The spool 62 illustratively includes a strut 65 bridging radially across the spool bed 61 to provide structural support and defining openings 67 to permit circulation of fluid 20 through the spool 62.

Returning briefly to Figure 1, as previously mentioned, the extraction probe 22 and the gas analysis module 24 are fluidly connected to define a gas circuit for circulation of gas therebetween. In the illustrative embodiment, the extraction probe 22 and the gas analysis module 24 are fluidly connected by transport conduit 64 including conduit segments 66, 68. The segment 66 is illustratively embodied as a supply segment for providing gas from the extraction probe 22 to the gas analysis module 24 and the segment 68 is embodied as a return segment for providing gas from the gas analysis module 24 to the extraction probe 22.

In the illustrative embodiment, the pump 54 is arranged fluidly along the supply segment 66 and provides a lower pressure level at the output of the extraction probe 22 (relative to the pressure of the gas cell 26), which may assist with extraction of dissolved gases. In some embodiments, the gas circuit may be formed substantially or entirely by the extraction probe 22 and gas analysis module 24 being fluidly connected with each other by direct connection and/or with little or no transport conduit 64. In some embodiments, the extraction probe 22 and gas analysis module 24 may be partly or wholly combined into a common module and/or arranged within a common housing for compact arrangement.

The gas circuit provides a circulation loop for communication of gas between the extraction probe 22 and the gas analysis module 24. In the illustrative embodiment, the gas circuit encourages the gas extracted from the fluid 20 to reach and maintain equilibrium with dissolved gases within the fluid 20. Such passive extraction and non-destructive analysis can avoid practical challenges with active sampling, such as fluid leaks, contamination, and waste materials, among others. Passive extraction does not rely on a precise determination of the extraction rate of the gas and thus reduces the need for factory calibration of each analyzer extraction rate. As mentioned above, the pump 54 illustratively assists circulation of the gas through the gas circuit and may assist extraction, but in some embodiments, circulation of the gas through the gas circuit may be provided by any suitable device(s), including but not limited to redundant pumps arrangements or arrangements without a pump such as convective and/or diffusive transport.

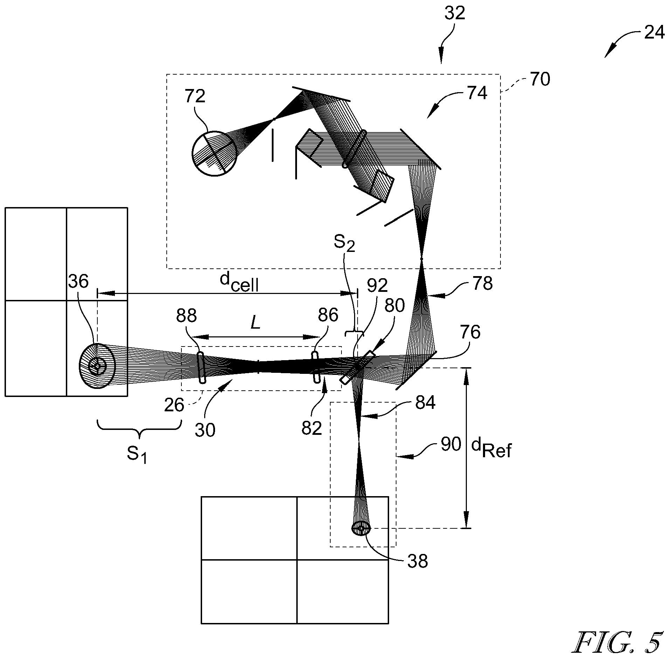

Referring now to Figure 5, a diagrammatic illustration of the gas analysis module 24 is shown. As mentioned above, the gas analysis module 24 illustratively includes the gas analysis device 32 arranged for conducting analysis of gas within the gas cell 26. The light source 34 of the gas analysis device 32 illustratively includes a light generation source 70. In the illustrative embodiment, the light generation source 70 includes an interferometer for modulating mid-IR light, for example, with a wavelength within a range of about 1 microns to about 50 microns (in some illustrative embodiments), about 2.5 microns to about 25 microns (in other illustrative embodiments), and about 2.5 to about 16 microns (in still other illustrative embodiments). The light generation source 70 also illustratively includes at least one light generator 72 for generating the mid-IR light and may include various relays, filters, and/or other conditioning devices (collectively indicated as 74) for providing suitable light for gas analysis. A non-limiting example of a suitable light generator 72 may include a glow bar (globar). The light source 34 illustratively includes a relay mirror 76 arranged to receive a beam of light 78 from the light generation source 70 and a beam splitter 80 arranged to receive the beam 78 from the relay mirror 76.

As shown in Figure 5, the gas analysis device 32 illustratively includes two optical channels as explained herein. The beam splitter 80 illustratively divides the beam 78 into two beams of light 82, 84 for spectrum analysis. The beam splitter 80 is illustratively embodied to have a beam- splitting ratio of 50:50 (50/50 splitter) dividing the beam 78 evenly into the two beams 82, 84, but in some embodiments, the beam splitter 80 may have other suitable beam-splitting ratios. In some embodiments, any suitable arrangement of relays, filters, splitters, and/or other conditioning devices may be employed to propagate light accordingly for gas analysis. Beams 82, 84 propagate through respective defined spaces for collection by detectors 36, 38.

In the illustrative embodiment as shown in Figure 4, analysis of the beams 82, 84 propagated through respective defined spaces can determine characteristics of the gas extracted from the fluid 20. The beam 82 illustratively propagates through the gas cell 26 for reception by detector 36. The beam 82 illustratively enters the gas cell 26 through a window 86, propagates through the cavity 30 for interaction with gas therein, and exits the gas cell 26 through another window 88. Light from the beam 82 exiting the gas cell 26 is received by the detector 36 for analysis. The gas within the cavity 30 affects the beam 82 in a manner such that the affected light received by detector 36 can indicate characteristics of the gas within the cavity 30. As explained below, the detector 36 can generate a signal related to the absorption spectrum of the gas within the cavity 30 based on the light received from beam 82.

In the illustrative embodiment, the gas within the cavity 30 absorbs energy from the beam 82 in the form of electromagnetic radiation. The remaining energy of beam 82 passes through the gas and is received by the detector 36 to generate a signal related to an absorption spectrum in the illustrative embodiment. The absorption spectrum of the relevant gas can include the fraction of incident radiation absorbed by the gas sample (in this instance, the gas within the cavity 30) over a range of wavelengths and/or frequencies of propagated light. By analysis of the light received by the detector 36 (for example, but without limitation, the wavelength and/or frequency thereof), the characteristics of the gas within the cavity 30 can be reliably determined. Moreover, characteristics of the dissolved gases within fluid 20 can be determined based on the characteristics of the gas within the cavity 30. In some embodiments, other analytical techniques and/or equipment may be used to determine gas characteristics. In some embodiments, additional gas analysis devices may be included in the gas cell to detect certain gases, such as hydrogen (H₂), oxygen (O₂), and/or nitrogen (N₂), and some of those additional gas analysis devices may use non-optical measurement principals that do not require gas interaction with light, such as resistive, capacitive, and/or thermo-conductive sensors, by way of example.

Accurate determination of the characteristics of gas within the cavity 30 (and ultimately the dissolved gases within fluid 20) should account for contaminants and/or artifacts. Common sources of artifacts includes constituents within the air contained in the gas analysis module 24 and/or constituents within the air in the vicinity of the transformer 10 that may enter the gas analysis module 24. For example, ambient air within the gas analysis module 24 can reduce the light received by the detector 36 even though it cannot enter into the cavity 30. Accordingly, reference information regarding the ambient environment can be useful in interpreting the light received by the detector 36. In the present disclosure, the terms "air" and "ambient air" are not intended to limit the gas constituents which can be considered, but may include any gas constituent, including constituents of the same species as the dissolved gases of interest in the fluid 20. By considering such reference information of ambient air, the characteristics of the gas within the cavity 30 (and by correspondence, the characteristics of the dissolved gases within the fluid 20) can be accurately determined by correction and/or calibration of the light received by the detector 36 (absorption spectrum). Such corrective approaches can reduce the need for purging, scrubbing, desiccants, relay adjustment, and/or other resource-laden or mechanically demanding techniques to achieve accurate results.

As shown in Figure 5, the beam 84 (split from the beam 82) illustratively propagates through a reference space 90 to provide characteristics of ambient air as reference information. The reference space 90 illustratively contains ambient gas (illustratively embodied as ambient air) which affects the beam 84 in a manner such that the affected light received by detector 38 can indicate characteristics of the ambient gas. The characteristics of the ambient gas can be used in interpreting the light received by detector 36. Analysis of the light received by the detector 36 in combination with the light received by the detector 38 can allow determination of characteristics of the gas within the cavity 30 (and, hence, the characteristics of the dissolved gases within the fluid 20) by reducing artifacts from the light absorbed by the ambient gas. Reduction of artifacts from the light absorbed by the ambient gas is illustratively achieved by consideration of the corresponding absorption spectra perceived by detectors 36, 38. In some embodiments, reference information may be obtained by any suitable technique and/or equipment.

In the illustrative embodiment as shown in Figure 5, the beam splitter 80 effectively provides a reference source point 92 for propagation of light through the defined spaces 30, 90. The reference source point 92 is illustratively represented as a single point on the beam splitter 80 for descriptive purposes. As shown in Figure 5, a propagation distance *d*ᵢ is illustratively defined between the reference source point 92 and each of the detectors 36, 38. A first propagation distance, referred to as a cell distance *d*_{cell}, is illustratively defined between the reference source point 92 and the detector 36. The cell distance *d*_{cell} illustratively corresponds to the propagation of the beam 82. A second propagation distance, referred to as a reference distance *d*_{Ref} is illustratively defined between the reference source point 92 and the detector 38. The reference distance *d*_{Ref} illustratively corresponds to the propagation of the beam 84. A third propagation distance, referred to as the cell body distance *L*, is illustratively defined between the first window 86 and the second window 88 delimiting the cavity 30 of the gas cell 26. In the illustrative embodiment, the distance (span) resulting from the subtraction of the cell body distance *L* from the cell distance *d*_{cell} (e.g., the span may include the sum of the distance between the reference source point 92 and the cavity 30, *S₁*, and the distance between the detector 36 and the cavity 30, *S₂*, as indicated in Fig. 5, either or both of which may contain ambient gas) is substantially equal to the reference distance *d*_{Ref} in such a way that the propagation distances in ambient air between the reference source point 92 and each of the detectors 36, 38 are substantially equal. In other embodiments, however, the propagation distances in ambient air between the reference source point 92 and each of the detectors 36, 38 may be different from each other and a correlation can be applied to equate their corresponding absorption spectra.

In some embodiments, the cell distance *d*_{cell} may be substantially equal to the sum of the reference distance *d*_{Ref} and the cell body distance *L*. In some embodiments, the propagation distances between the reference source point 92 and each of the detectors 36, 38 may be substantially equal. In some embodiments, the propagation distances may be different from each other and a correlation can be applied to equate their corresponding absorption spectra.

In the illustrative embodiment, the light source 34 provides the beam of light 78 for division into beams 82, 84 for respective propagation through each of the cavity 30 and reference space 90. Thus, the light source 34 illustratively provides each of beams 82, 84 simultaneously from the same source for use in two optical channels; one channel analyzing light propagated through the gas cell 26, and another channel analyzing light propagated through the reference space 90. The dual channel arrangement using the same source of light can promote uniformity between the spectral characteristics of the channels and decrease adjustable parameters (e.g., moving optics, pressure/temperature modulation of gas samples) and/or the use of commodities (e.g., purge gas, desiccants, scrubbers) in obtaining reliable readings.

Devices, systems, and methods of the present disclosure can be advantageous for remote operation where commodities and/or maintenance availability is of concern. Moreover, arrangements of the present disclosure can account for unexpected and/or unknown contaminants, even without identifying the exact contaminant. In some embodiments, the reference information of the ambient gas may not identify one or more of the substances in the gas analysis module 24 and/or located between the light generator 72 and detectors 36, 38. However, the reference information of the unidentified substance can still be considered in accurately determining the characteristics of the gas within the cavity 30.

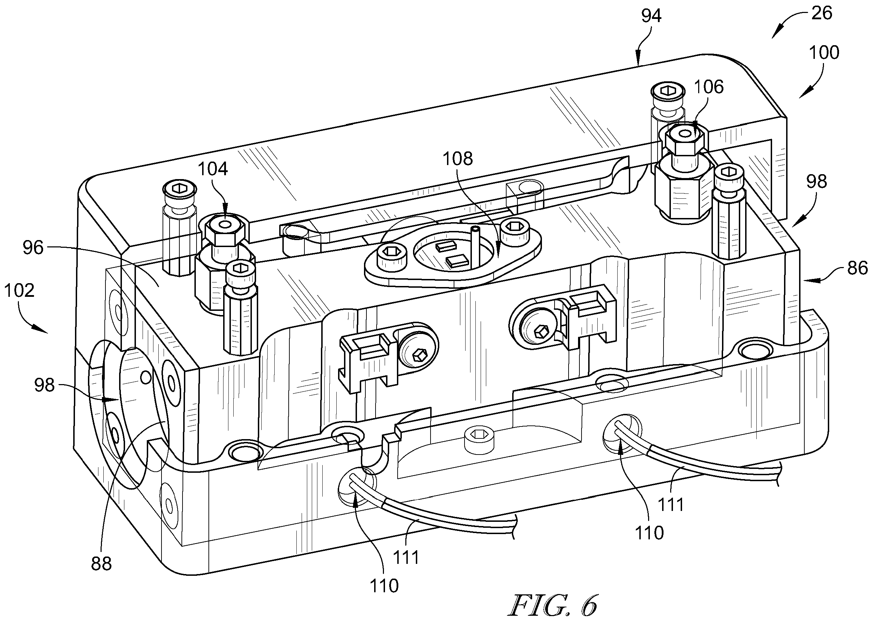

Referring now to Figure 6, an illustrative embodiment of the gas cell 26 is shown. The gas cell 26 illustratively includes a housing 94, which is shown partially cutaway (and semi- transparent) to reveal a cell body 96 that defines the cavity 30 therein (the cell body 96 being an illustrative embodiment of the cell body 28 of Figure 1). The cell body 96 illustratively includes openings 98 penetrating through the cell body 96 on opposite ends 100, 102 to connect with the cavity 30. Each opening 98 is enclosed by a respective one of the windows 86, 88. The cell body 96 illustratively includes gas ports 104, 106 that each penetrate through the housing 94 and fluidly connect with the cavity 30 to form a portion of the gas circuit to communicate gas with the extraction probe 22.

The gas port 104 is illustratively embodied as an inlet port (relative to the gas cell 26) for receiving gas from the extraction probe 22 and the gas port 106 is embodied as an outlet port for sending gas to the extraction probe 22. The cell body 96 illustratively includes pressure and temperature sensor ports 108 for insertion of pressure and temperature sensors 122, 124 (shown in Figure 1) to monitor the conditions within the cavity 30. A cell heater 110 including electrical leads 111 is illustratively connected with the cell body 96 within the housing 94 to provide temperature control of the cavity 30.

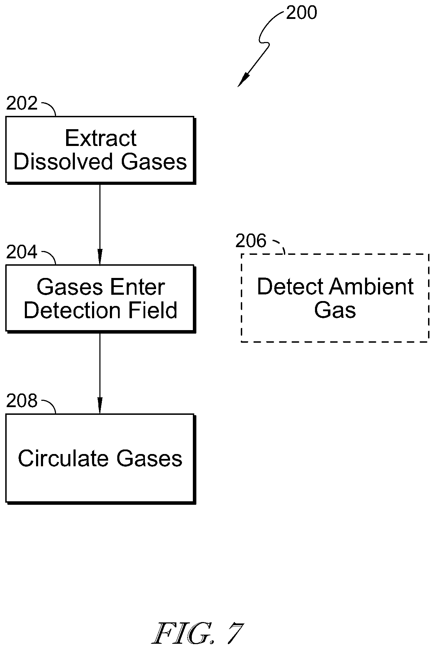

Referring to Figure 7, an illustrative flow diagram is shown. A process 200 for determining characteristics of gases is described relative to boxes 202-208. In box 202, dissolved gases are illustratively extracted from fluid 20 of the transformer 10. In the illustrative embodiment, the dissolved gases are extracted by permeation into the extraction probe 22 to enter the gas circuit. The process illustratively proceeds from box 202 to box 204.

In box 204, extracted gas illustratively enters a detection field. In the illustrative embodiment, the extracted gas enters the detection field as it circulates through the gas cell 26 and light is propagated through the extracted gas for reception by the detector 36. In embodiments in which reference information is used for correction, in box 206, the characteristics of ambient gases are detected. In the illustrative embodiment, the second channel of the gas analysis module 24 propagates light through the reference space 90 and the ambient gas therein for reception by the detector 38. The process proceeds from box 204 to box 208.

In box 208, gas within the detection field circulates out of the detection field. In the illustrative embodiment, gas within the gas cell 26 is circulated through the gas circuit to return to the extraction probe 22. The circulation of the gas within the gas circuit promotes nondestructive testing and enables equilibrium between gas in the gas circuit and dissolved gas within the fluid 20.

Returning briefly to Figure 1, operation of the gas analysis system 12 and the various methods and functions described herein is illustratively governed by a control system 112. The control system 112 illustratively includes a processor 114, memory device 116, and communications circuitry 118 in communication with each other. The memory device 116 stores instructions for execution by the processor 114 to conduct operations of the gas analysis system 12. In the illustrative embodiment, the instructions include at least one algorithm for conducting the disclosed operations, but in some embodiments, the instructions may include any of look up tables, charts, and/or other reference material. The communications circuitry 118 illustratively includes various circuitry arranged to send and receive communication signals between the control system 112 and various components as directed by the processor 114. It will be appreciated that the communications circuitry 118 also allows the control system 112 to communicate with other devices, including remote devices, and along various communications networks, such that the gas analysis system 12 (as well as the transformer 10) can be connected to and form part of the Internet of Things. As a result, various components of the gas analysis system 12 may be sensed and/or controlled remotely across existing network infrastructure.

The control system 112 is illustratively arranged in communication with the gas analysis module 24 and the pump 54 through communication links 120 to communicate signals to govern their operation. Communication links 120 illustratively include hardwired connections, but in some embodiments may include any of hardwired and wireless connections, and/or combinations thereof. In the illustrative embodiment, the control system 112 is in communication with each of the light source 34, the detectors 36, 38, gas cell temperature and pressure sensors 122, 124 through individual links 120, but in some embodiments, the control system 112 may be in communication with components of the gas analysis module 24 by one or more shared links 120. The control system 112 illustratively performs spectrum analysis of the light received by the detectors 36, 38 and determines the characteristics of the gas within the cavity 30 and the corresponding characteristics of the dissolved gas within the fluid 20.

As shown in Figure 1, the transformer 10 illustratively includes a pump 126 arranged to circulate fluid 20 within the housing 14. Circulating the fluid 20 can assist in providing uniform distribution of dissolved gases and can assist in extracted gases reaching accurate equilibrium faster than with stagnant fluid conditions. In the illustrative embodiment, the pump 126 is a thermal pump circulating the fluid 20 by convective movement. In other embodiments, any suitable device for circulating the fluid 20 may be used, including, for example, a displacement pump and/or an agitator. In the illustrative embodiment, the control system 112 is in communication with the pump 126 to govern operation of the pump 126.

In the illustrative embodiment, the control system 112 is embodied to govern operations of all components of the gas analysis system 12. In some embodiments, the control system 112 may govern operation of other systems of the transformer 10. In some embodiments, the control system 112 may include multiple processors, memory devices, and/or communications circuitry that may have any suitable arrangement including but not limited to dedicated and partly or wholly shared arrangements. In some embodiments, another control system 112 may be dedicated to govern operation of the gas analysis module 24 and the remainder of the gas analysis system 12 may be governed by the control system 112.

As mentioned above, the extraction probe 22 may include a suitable permeable material, for example, fluoropolymers. Suitable gas-permeable materials may include, for example, but without limitation, amorphous fluoroplastics. such as Teflon^{®} AF and/or Chemours^{®} AF, as marketed by Professional Plastics, Inc. (under affiliation and/or with permission from DuPont^{®}), with typical properties as shown in the table below:

| Typical Properties of Teflon^{®} AF | |
|---|---|
| Optical Clarity | Clear: >95% |
| Upper Use Temperature, °C (°F) | 285 (545) |
| Thermal Stability, °C (°F) | 360 (680) |
| Thermal Expansion (linear), ppm/°C | 80 |
| Water Absorption | No |
| Weatherability | Outstanding |
| Flame Resistant LOI, % | 95 |
| Tensile Modulus, MPa (psi) | 950-2150 (137, 786-311, 832) |
| Creep Resistance | Good |
| Solubility | Selected solvents |
| Resistance to Chemical Attack | Excellent |
| Surface-Free Energy | Low |
| Refractive Index | 1.29-1.31 |
| Dielectric Constant | 1.89-1.93 |

Non-limiting examples may include Teflon^{®} AF 1600 and/or Teflon^{®} AF 2400 (and/or Chemours^{®} AF 1600 and/or AF 2400) having typical properties as described within the table below:

### Typical Property Data for Teflon^{®} AF Amorphous Fluoroplastics

| **Property** | | **ASTM Method** | **Unit** | **Grade** | |
|---|---|---|---|---|---|
| | | | | **1600** | **2400** |
| **Electrical** | | | | | |
| Dielectric Constant | | D150 | | 1.93 | 1.90 |
| Dissipation Factor | | D150 | | 0.0001-0.0002 | 0.0001-0.0003 |
| Dielectric Strength | | D149 | kV/0.1 mm | 2.1 | 1.9 |

| **Optical** | | | | | |
|---|---|---|---|---|---|
| Optical Transmission | | D1003 | % | >95 | >95 |
| Refractive Index | | D542 | | 1.31 | 1.29 |
| ABBE Number | | | | 92 | 113 |

| **Mechanical** | | | | | |
|---|---|---|---|---|---|
| Yield Strength | | | MPa | | |
| | | | 23°C (73°F) | 27.4 ± 1.0 | 26.4 ± 1.9 |
| | | | 150°C (302°F) | 6.7 ± 5.9 | |
| | | | 220°C (428°F) | | 8.7 ± 4.0 |
| Tensile Strength | | D638 | MPa | | |
| | | | 23°C (73°F) | 26.9 ± 1.5 | 26.4 ± 1.9 |
| | | | 150°C (302°F) | 7.7 ± 6.1 | |
| | | | 220°C (428°F) | | 4.2 ± 1.8 |
| Elongation at Break | | D638 | % | | |
| | | | 23°C (73°F) | 17.1 ± 5.0 | 7.9 ± 2.3 |
| | | | 150°C (302°F) | 89.3 ± 13.1 | |
| | | | 220°C (428°F) | | 84 ± 4.1 |
| Tensile Modulus | | D638 | GPa | 1.6 | 1.5 |
| Flexural Modulus | | D790 | GPa | | |
| | | | 23°C (73°F) | 1.8 ± 0.1 | 1.6 ± 0.1 |
| | | | 150°C (302°F) | 1.0 ± 0.1 | |
| | | | 220°C (428°F) | | 0.7 ± 0.1 |
| Hardness | | | | | |
| | Rockwell | D785 | 23°C (73°F) | 103 | 97.5 |
| | Durometer | D1706 | Shore D | | |
| | | | 23°C (73°F) | 77 | 75 |
| | | | 150°C (302°F) | | |
| | | | 220°C (428°F) | 70 | 65 |
| Impact Strength | | Notched Izod | N | - | - |
| Deflection Temperature | | D648 | °C (°F) | | |
| | (66 psi) | | | 156(313) | 200 (392) |
| | (264 psi) | | | 154 (309 | 174 (345) |

| **Chemical** | | | | | |
|---|---|---|---|---|---|
| Contact Angle with Water | | D570 | Degrees | 104 | 105 |
| Critical Surface Energy | | | Dynes/cm | 15.7 | 15.6 |
| Taber Abrasion | | | cc/2000 cycles | 0.107 | 0.2 |
| Chemical Resistance | | | | | |
| | Water Absorption | | % | <0.01 | <0.01 |
| Gas Permeability | | | | | |
| | H₂O | | Barrer | 1142 | 4026 |
| | O₂ | | Barrer | 340 | 990 |
| | N₂ | | Barrer | 130 | 490 |
| | CO₂ | | Barrer | | 2800 |

| **Other** | | | | | |
|---|---|---|---|---|---|
| T_{g} | | D3418 | °C (°F) | 160 (320) ± 5 | 240 (464) ± 10 |
| Specific Gravity | | D792 | | 1.78 | 1.67 |
| Melt Viscosity | | D3835 | Pa s | 2657 at 250°C, 100 s⁻¹ | 540 at 350°C, 100 s⁻¹ |
| Volume Coefficient of Thermal Expansion | | E831 | ppm/°C | 260 | 301\| |

In some embodiments, any suitable materials for gas-permeable, liquid-resistant extraction of dissolved gases from transformer fluid may be applied.

The present disclosure includes devices, systems, and methods for oil and gas management for dissolved gas analyzers for use in transformer monitoring. The devices, systems, and methods of the present disclosure may include detecting dissolved gases in insulating oil of electrical equipment using gas equilibrium theory. Equilibrium can be achieved relative to the solubility of a gas in a transformer fluid 20, such as mineral oils, ester-based oils, or other insulation fluids, at a given temperature and for a given partial vapor pressure of a gas. Gas solubility can be described with quantities such as Ostwald coefficients of gas solubility that are specific to the type of fluid and to each gas constituent and may have temperature dependency. Gas solubility coefficients can be used to relate the partial pressure of gas in the gas cell with the concentration of dissolved gas in oil. The extracted gases being in equilibrium with the dissolved gases in oil may provide more accurate readings without requiring precise knowledge of extraction rates. In some embodiments, the extraction probe 22 of the present disclosure may comprise at least one ring of highly gas permeable tubing that is not permeable to liquid. In some embodiments, the extraction probe 22 may be connected to a closed-circulation system. The closed-circulation system may include one or more pumps for gas circulation and a gas cell, for example, gas cell 26, for analytical measurement of the gas.

The present disclosure includes devices, systems, and methods adapted to monitor the health of a transformer by measuring dissolved gases within insulating oil of the transformer. For example, the concentration of specific gases can give indications of specific aspects of the operation of the transformer. Direct oil sampling and analysis of dissolved gases contained in transformer oil use active extraction of the gases and active measurement technics that consume the gases through the analyses. They are often implemented by circulating and/or conditioning oil samples outside the transformer in an oil circuit and may present a risk of oil leakage in case of breakage of the oil circuit. By contrast, embodiments of the devices, systems, and methods of the present disclosure permit online measurement with high accuracy and without active extraction. In some of the disclosed embodiments, oil containing the dissolved gases is circulating around highly permeable material tube within a fluid chamber 46 communicating fluidly with the transformer 10 through the pipe extension 42. In some embodiments, the oil circulation around the permeable tube may be generated by pump, propeller and/or other mechanical systems and/or using thermally induced convection. Gases contained in oil can pass through permeable material to reach the gas phase loop. The permeable material properties can assist in obtaining equilibrium between gases in the liquid and gases in the gas phase loop. The gas loop may include a gas cell with optical inlet and outlet allowing examination of the gases by optical analyzer.

Devices, systems, and methods of the present disclosure may include highly permeable fluoropolymer tubing, such as Teflon AF family of amorphous fluoroplastics, by way of example. Highly gas permeable material can promote gas equilibrium and can improve measurement response time. The tubing may be rolled to form one or more turns of a coil. Devices, systems, and methods of the present disclosure may include circulation of the transformer fluid (e.g., oil) around this coil. A structural ring may support the tubing. According to the present disclosure, the fluoropolymer tubing may be connected to a gas circulating loop. The gas circulating loop may include one or more pumps to enhance reliability. In some embodiments, stainless steel tubing may transport gas to a gas cell for analysis. In some embodiments, a spectrometer may perform analysis of the gases. In some embodiments, in-oil sensors may be used for H₂ and/or H₂O measurement. Devices, systems, and methods of the present disclosure may include passive extraction of dissolved gases and measurement, in lieu of active principles for gas separation and measurement. In some embodiments, the present disclosure may include transport of extracted gases without a carrier medium (e.g., a carrier gas). In some embodiments, a lower pressure may be formed within the extraction probe 22, relative to the pressure within the gas cell 26 to assist with extraction of dissolved gases.

Devices, systems, and methods of the present disclosure can be used in transformer monitoring and/or specifically in monitoring of dissolved gases analysis in transformer fluid such as oil. For gas phase analysis, gases can be extracted from the transformer oil. Measurement of the gases can require a complex system for analysis, and in some embodiments, the gas sample can be transported to a gas analyzer. The devices, systems, and methods of the present disclosure can be helpful in avoiding transporting the transformer oil itself to an analyzer, which can present a risk of oil leakage in case of tubing breakage.

Use of passive measurement and passive extraction of the gases can simplify the calibration and installation of gas analysis systems. Use of high porosity and/or highly permeability material can help to reach equilibrium between gases in oil and gases in the sample gas phase. Using gases equilibrium, without requiring new gases to be sampled, can reduce risk of contamination of the oil. Use of a lower pressure (relative to the pressure within the gas cell) in the gas sampling probe can reduce the response time of the systems. The use of multiple transport pumps can help to reduce risk of failure. In some embodiments, measurement of H₂ may be conducted in gas phase to reduce the cost. In some embodiments, measurement of O₂, H₂, and/or N₂ may be performed optically and/or with non-optical sensors. In some embodiments, O₂ can be measured by paramagnetic analyzer. In some embodiments, gas leak detection may be performed by monitoring the presence of CO₂ or H₂O with the gas cell, whether by direct and/or indirect sampling. The present disclosed devices, systems, and methods may involve advanced analyses and identification of interferent and outlier.

The present disclosure includes devices, systems, and methods for dual channel optical gas analyzers for compensation of ambient air constituents. Spectrometers can be used to measure light absorption spectra of gases. When gases of interest in a sample under observation are also present in ambient air (e.g., air either in the analyzer and/or around the sampling system) or when other gases in ambient air might interfere with the measurement of the gases of interest, spectrometers often must be purged, for example, with a purified gas to determine the contribution due to the absorption of only the gases of interest in the gas sample. The present disclosure includes devices, systems, and methods to reduce and/or remove the need for conditioning of the air in the analyzer or around the sampling system. The present disclosure includes spectrometers with two measurement channels. One channel can receive light propagating through ambient air and through a sampling gas cell. The transmitted light is then detected by a photodetector which generates an electrical signal that is digitized using an analog to digital converter. Another channel receives light propagating through ambient air only. Unlike in the first channel, the light of this second channel is not propagating through the sampling gas cell. The gas absorption contribution to the transmitted light in this second channel is related to ambient air constituents. The transmitted light of this second channel is detected by a second photodetector which generates an electrical signal that is digitized using a second analog to digital converter.

Devices, systems, and methods within the present disclosure may include light sources that split the light (e.g., by beam splitter, light divider, and/or any other suitable light splitting technique), a gas cell that may contain one or many gases of interest, components to insert gases into the gas cell, a first detector measuring the light transmitted through the gas cell and through ambient air, a second detector measuring the light transmitted only through ambient air, a processor to determine the concentration of one or more gases present in the sampling gas cell from the first channel signal, and remove interferences and/or contribution of gases in ambient air of the first channel based on the ambient air signal recorded from the second channel.

In some embodiments, a light source may be modulated by an interferometer. The light source may be divided in two different beams by a 50/50 Wedged ZnSe Beamsplitter. One of the beams may propagate through the gas cell and may reach the gas cell detector. The other beam may be directed towards a reference detector, to sense the ambient air composition only. The propagation distance in ambient air can be adjusted for both beams. The adjustment can be performed in a manner such that both the light transmitted by the gas cell and reaching the first detector and the light reaching the reference detector of the second channel propagate through similar distances in ambient air. In some embodiments, it may be assumed that ambient air composition in the instrument is homogeneous, and the light absorption due to the gases from ambient air should be the proportional to the gases concentration as well as to the respective propagation distance of both channels.

In some embodiments, the gas cell may be a closed container with one inlet and one outlet to fluidly connect to form a gas circulation loop. The light from the interferometer can enter the gas cell from one side and exit through the other side to the gas cell detector. The gas cell can be temperature controlled by a cartridge heater. The pressure and temperature of the gases in the gas cell can be measured and used as input parameters to the calculation of gases concentrations.

The present disclosure includes devices, systems, and methods in which the need for a purging system can be reduced and/or removed. Reducing and/or removing the need for a purging system can be an advantage when an analyzer is located in remote areas and purging systems are not available and/or are costly to install and operate. Concentration of gases in the gas cell that may also be present ambient air can be determined without purge, scrubber, desiccant and/or analyzer sealing. Other ambient air gases which have absorption signatures that may interfere with the determination of the gases concentration in the gas cell may also be compensated without purge, scrubber, desiccant and/or sealing. With the teachings of the present disclosure, the gases in ambient air can be measured simultaneously with the gases in the gas cell if desired, as opposed to calibration methods where only one channel can be used. Single channel calibration may perform reference background measurement taken apart from and/or without the gases of interest in the gas cell. The devices, systems, and methods of the present disclosure can provide an advantage when ambient air composition varies over time. The devices, systems, and methods of the present disclosure can include calibration for spectral intensity of the source, and calibration for the spectral characteristics of optical components that are common to both the first and second channels.

The present disclosure is used in the field of transformer monitoring by analysis of dissolved gases. The teachings of the present disclosure are generally applicable to other fields where optical methods require purge, scrubber, desiccant and/or sealing in order to calibrate, remove, and/or correct for ambient air constituents, but this does not form part of the claimed invention.

The devices, systems, and methods of the present disclosure can provide an alternative to systems taking reference measurements using only one detector, by removing the gases of interests from the gas cell and/or bypassing the gas cell.

Measuring low concentration gases by spectroscopy with accuracy can be challenging, particularly when the same gases or other interfering gases are present in ambient air, either in the analyzer or around the sampling system. Concentration of these gases in ambient air and/or the relative propagation distance of the light in ambient air could be non- negligible compared to the concentration of the gases in the gas cell and the propagation distance in the gas cell. Furthermore, the concentration of these gases in ambient air may vary with time, and unexpected gases can appear in ambient air in some sites. Pressure and temperature of the ambient air may differ from the pressure and temperature of the gas sample in the gas cell.

To remove the contribution of ambient air gases, analyzers are often purged with purified gases (by way of example, the MB3000 spectrometer marketed by ABB Inc., includes a purging option). Purging can require bottles of purified gases, like Nitrogen, and/or a purified gas generator. Purge air is often dried to remove humidity, which can be a significant interferent in some instances, and CO₂ is often removed as well with a scrubber. In other systems where a purge is not possible and/or desirable, desiccants and/or scrubbers are used to remove humidity and/or other gases, but must be replaced or regenerated after some time. Other exemplary techniques can include moving relay mirrors to the gas cell in and out of the first channel in order to bypass the gas cell and direct the light to the detector to take background measurement. The relay optics can be designed such that the propagating distance in air with and without the relay optics is the same. Still other exemplary techniques can include using a scrubber to remove the gas component of interest from the gas cell after measuring the gas sample with the gas component of interest and inferring its concentration by the comparison of those two alternate measurements. Still other techniques may vary the pressure and/or the temperature of the gas sample to discriminate its composition over ambient air composition. In cases where the purge gas is supplied from an exhaustible source, such as a bottle, the exhaustible source will need to be refilled and/or changed at periodic maintenance intervals. Purge generators can be costly equipment that can require maintenance as well. Scrubbers and desiccants also require maintenance. Thus, the purge-based systems can increase the cost of operating spectrometers.

As mentioned above, the present disclosure can include reducing and/or removing the need for a purging system, desiccants, scrubbers and/or instrument sealing. Accordingly, the devices, systems, and methods of the present disclosure can reduce installation and/or maintenance costs related to the spectrometer, and can enable solutions for remote sites where purging systems are not available and/or maintenance cannot be performed frequently due to cost and/or safety issues. In some embodiments, the devices, systems, and methods of the present disclosure do not require moving optics and/or sample gas pressure modulation, and the ambient air constituents can be measured simultaneously with the gas cell constituents.

Since spectrometers using certain teachings of the present disclosure can measure spectra of ambient air, they may also detect and/or compensate for unexpected gases present in the ambient air, as opposed to scrubbers that are designed for specific constituents. Devices, systems, and methods of the present disclosure may be used to detect other defects around the transformer, for example but without limitation, detection of insulation gas leaks, such as SF₆. By measuring and removing ambient air absorption, the devices, systems, and methods of the present disclosure can reduce sensitivity to ambient air compositions. The composition of the air inside the optical analyzer and/or around the sampling system may not need to be controlled by use of purge system, desiccants, scrubbers and/or instrument sealing.

In some embodiments, the devices, systems, and methods of the present disclosure may use factory calibration to characterize the difference of light propagating distances in air between first and second channels. In some embodiments, the devices, systems, and methods of the present disclosure may use factory calibration of the system to measure the spectral response of the first and second detectors as well as spectral response of optical components not common to first and second channel. In some embodiments, the devices, systems, and methods of the present disclosure may use factory calibration to characterize the spectral response and/or instrument line shape of the first and second channels in order to improve the compensation of air constituents in the first channel using the second channel. Factory calibration may include purge of the analyzer. The present disclosure include techniques developed to adjust the position of system components (mirrors, lenses, detectors, etc.) to minimize the difference of light propagating distance in air between first and second channel. In some embodiments, one or more algorithms may be used to compensate for the ambient constituents of the first channel using the second channel signal.

While certain illustrative embodiments have been described in detail in the figures and the foregoing description, such an illustration and description is to be considered as exemplary and not restrictive in character, it being understood that only illustrative embodiments have been shown and described and that all changes and modifications that come within the scope of the claims are desired to be protected. There are a plurality of advantages of the present disclosure arising from the various features of the methods, systems, and articles described herein. It will be noted that alternative embodiments of the methods, systems, and articles of the present disclosure may not include all of the features described yet still benefit from at least some of the advantages of such features. Those of ordinary skill in the art may readily devise their own implementations of the methods, systems, and articles that incorporate one or more of the features of the present disclosure and that fall within the scope of the appended claims.

## Claims

1. A transformer (10), comprising:
at least one electrical winding (18);
a fluid system including fluid (20) for insulating the at least one electrical winding (18); and
a gas analysis system (12) for determining characteristics of gas dissolved in the fluid (20) of the fluid system, the gas analysis system (12) including an extraction coil (22) and a gas cell (26) for analysis of gas, the extraction coil (22) arranged in contact with the fluid (20) and including a gas-permeable material for receiving dissolved gas from the fluid (20), the extraction coil (22) and the gas cell (26) fluidly communicating to form a gas circulation loop for circulating gas,
wherein the gas cell (26) is arranged for determining characteristics of gas extracted from the fluid (20),
**characterized in that** the gas analysis system (12) includes an extraction module (50) for mounting the extraction coil (22) within a housing (14) of the transformer (10), the extraction module (50) comprising:
a mounting frame (52) including an engagement wall (56) and a probe arm (58) extending from the engagement wall, wherein the probe arm (58) includes a spool (62) having the extraction coil (22) looped around the spool (62).

2. The transformer (10) of claim 1, wherein the gas circulation loop further includes a transport conduit (64) fluidly coupled with each of the extraction coil (22) and the gas cell (26) to transport gas received from the fluid (20) to the gas cell (26) for analysis.

3. The transformer (10) of claim 2, further comprising a motive pressure source (54) to circulate gas through the transport conduit (64).

4. The transformer (10) of any preceding claim, wherein the extraction coil (22) is formed as a conduit having an inner volume for receiving gas permeating through the gas-permeable material.

5. The transformer (10) of claim 4, wherein a gas species that is both within the inner volume and dissolved in the fluid (20) is in equilibrium.

6. The transformer (10) of any preceding claim, wherein the gas-permeable material includes a fluoropolymer.

7. The transformer (10) of claim 6, wherein the gas-permeable material includes a fluoroplastic having at least one of: a yield strength within the range of about 26 MPa to about 29 MPa at about 73 °F, a yield strength within the range of about 0.5 MPa to about 13 MPa at about 302 °F, a yield strength within the range of about 4 MPa to about 13 MPa at about 428 °F, a tensile strength within the range of about 24 MPa to about 29 MPa at about 73 °F, a tensile strength within the range of about 1 MPa to about 15 MPa at about 302 °F, and a tensile strength within the range of about 3 MPa to about 7 MPa at about 428 °F.

8. The transformer (10) of any preceding claim, wherein the extraction coil (22) includes a number of coil loops each permitting dissolved gas to permeate therein.

9. The transformer (10) of any preceding claim, wherein the gas cell (26) has a cavity for receiving gas for analysis and is part of a gas analyzer (24) which further includes a light source (34) and at least one light detector (36, 38) for receiving light from the light source (34).

10. The transformer (10) of claim 9, wherein the light source (34) is arranged to pass light from one side of the gas cell (26) through gas within the cavity (30) of the gas cell (26) to another side of the gas cell (26), and the at least one light detector (36) is arranged on the another side to receive light from the light source (34).

11. The transformer (10) of any proceeding claim, wherein:
the gas circulation loop includes a transport conduit (64) fluidly coupled with each of the extraction coil (22) and the gas cell (26) to transport gas between the extraction coil (22) and the gas cell (26) for analysis,
the transport conduit (64) includes a pump (54) fluidly coupled with the extraction coil (22) to circulate gas through the transport conduit (64), and
the pump (54) is fluidly coupled with the extraction coil (22) to provide a lower pressure at an output of the extraction coil (22) relative to a pressure within the gas cell (26).

12. The transformer (10) of claim 11, wherein:
the pump (54) is mounted to the mounting frame (52).

13. The transformer (10) of claim 12, wherein:
the extraction coil (22) is looped around the spool (62) to form a number of coil turns having a successively stacked arrangement for exposure to the fluid (20).

14. The transformer (10) of claim 13, wherein:
the spool (62) includes an annular spool bed (61) for receiving the extraction coil (22) wrapped thereon, wherein the annular spool bed (61) defines openings (63) extending through the annular spool bed (61) that permits the fluid (20) to contact interior portions of the extraction coil (22).

15. The transformer (10) of claim 14, wherein:
the spool (62) includes a strut (65) bridging radially across the annular spool bed (61) to provide structural support and defining openings (67) to permit circulation of the fluid (20) through the spool (62).

## Patentansprüche

1. Transformator (10), Folgendes umfassend:
mindestens eine elektrische Wicklung (18);
ein Fluidsystem, das Fluid (20) zum Isolieren der mindestens einen elektrischen Wicklung (18) umfasst; und
ein Gasanalysesystem (12) zur Bestimmung von Charakteristika von in dem Fluid (20) des Fluidsystems gelöstem Gas, wobei das Gasanalysesystem (12) eine Extraktionsspule (22) und eine Gaszelle (26) zur Analyse von Gas umfasst, wobei die Extraktionsspule (22) in Kontakt mit dem Fluid (20) angeordnet ist und ein gasdurchlässiges Material zur Aufnahme von gelöstem Gas aus dem Fluid (20) umfasst, wobei die Extraktionsspule (22) und die Gaszelle (26) in Fluidverbindung stehen, um eine Gaszirkulationsschleife für Gas zu bilden,
wobei die Gaszelle (26) zur Bestimmung von Charakteristika von aus dem Fluid (20) extrahiertem Gas angeordnet ist,
**dadurch gekennzeichnet, dass** das Gasanalysesystem (12) ein Extraktionsmodul (50) für die Montage der Extraktionsspule (22) innerhalb eines Gehäuses (14) des Transformators (10) umfasst, wobei das Extraktionsmodul (50) Folgendes umfasst:
Montagerahmen (52), der eine Eingriffswand (56) und einen sich von der Eingriffswand erstreckenden Sondenarm (58) umfasst, wobei der Sondenarm (58) eine Spule (62) umfasst, wobei die Extraktionsspule (22) um die Spule (62) gewunden ist.

2. Transformator (10) nach Anspruch 1, wobei die Gaszirkulationsschleife ferner eine Transportleitung (64) umfasst, die mit der Extraktionsspule (22) und der Gaszelle (26) fluidisch gekoppelt ist, um aus dem Fluid (20) erhaltenes Gas zur Analyse zur Gaszelle (26) zu befördern.

3. Transformator (10) nach Anspruch 2, ferner umfassend eine Antriebsdruckquelle (54), um Gas durch die Transportleitung (64) zu zirkulieren.

4. Transformator (10) nach einem der vorhergehenden Ansprüche, wobei die Extraktionsspule (22) als eine Leitung mit einem Innenvolumen zur Aufnahme von Gas ausgebildet ist, das durch das gasdurchlässige Material hindurchtritt.

5. Transformator (10) nach Anspruch 4, wobei eine Gasspezies, die sich sowohl im Innenvolumen befindet als auch in der Flüssigkeit (20) gelöst ist, im Gleichgewicht ist.

6. Transformator (10) nach einem der vorhergehenden Ansprüche, wobei das gasdurchlässige Material ein Fluorpolymer beinhaltet.

7. Transformator (10) nach Anspruch 6, wobei das gasdurchlässige Material einen Fluorkunststoff mit mindestens einem der folgenden Merkmale aufweist: eine Streckgrenze im Bereich von etwa 26 MPa bis etwa 29 MPa bei etwa 73 °F, eine Streckgrenze im Bereich von etwa 0,5 MPa bis etwa 13 MPa bei etwa 302 °F, eine Streckgrenze im Bereich von etwa 4 MPa bis etwa 13 MPa bei etwa 428 °F, eine Zugfestigkeit im Bereich von etwa 24 MPa bis etwa 29 MPa bei etwa 73 °F, eine Zugfestigkeit im Bereich von etwa 1 MPa bis etwa 15 MPa bei etwa 302 °F und eine Zugfestigkeit im Bereich von etwa 3 MPa bis etwa 7 MPa bei etwa 428 °F.

8. Transformator (10) nach einem der vorhergehenden Ansprüche, wobei die Extraktionsspule (22) eine Anzahl von Spulenwindungen umfasst, die jeweils das Eindringen von gelöstem Gas ermöglichen.

9. Transformator (10) nach einem der vorhergehenden Ansprüche, wobei die Gaszelle (26) einen Hohlraum zur Aufnahme von zu analysierendem Gas aufweist und Teil eines Gasanalysators (24) ist, der ferner eine Lichtquelle (34) und mindestens einen Lichtdetektor (36, 38) für die Aufnahme von Licht von der Lichtquelle (34) umfasst.

10. Transformator (10) nach Anspruch 9, wobei die Lichtquelle (34) angeordnet ist, um Licht von einer Seite der Gaszelle (26) durch Gas innerhalb des Hohlraums (30) der Gaszelle (26) zu einer anderen Seite der Gaszelle (26) zu leiten, und der mindestens eine Lichtdetektor (36) auf der anderen Seite angeordnet ist, um Licht von der Lichtquelle (34) zu empfangen.

11. Transformator (10) nach einem der vorhergehenden Ansprüche, wobei:
die Gaszirkulationsschleife eine Transportleitung (64) aufweist, die fluidisch mit der Extraktionsspule (22) und der Gaszelle (26) gekoppelt ist, um Gas zwischen der Extraktionsspule (22) und der Gaszelle (26) zur Analyse zu befördern,
die Förderleitung (64) eine Pumpe (54) umfasst, die mit der Extraktionsspule (22) fluidisch gekoppelt ist, um Gas durch die Förderleitung (64) zu zirkulieren, und
die Pumpe (54) mit der Extraktionsspule (22) fluidisch gekoppelt ist, um einen niedrigeren Druck an einem Ausgang der Extraktionsspule (22) relativ zu einem Druck innerhalb der Gaszelle (26) bereitzustellen.

12. Transformator (10) nach Anspruch 11, wobei:
die Pumpe (54) am Montagerahmen (52) montiert ist.

13. Transformator (10) nach Anspruch 12, wobei:
die Absaugspule (22) um die Spule (62) gewunden ist, um eine Anzahl von Spulenwindungen mit einer aufeinander folgenden Stapelanordnung zur Beaufschlagung mit dem Fluid (20) zu bilden.

14. Transformator (10) nach Anspruch 13, wobei:
die Spule (62) ein ringförmiges Spulenbett (61) zur Aufnahme der darauf gewickelten Extraktionsspule (22) umfasst, wobei das ringförmige Spulenbett (61) Öffnungen (63) definiert, die sich durch das ringförmige Spulenbett (61) erstrecken, die es dem Fluid (20) ermöglichen, mit Innenabschnitten der Extraktionsspule (22) in Kontakt zu treten.

15. Transformator (10) nach Anspruch 14, wobei:
die Spule (62) eine Strebe (65) umfasst, die sich radial über das ringförmige Spulenbett (61) erstreckt, um eine strukturelle Unterstützung bereitzustellen und Öffnungen (67) zu definieren, um die Zirkulation des Fluids (20) durch die Spule (62) zu ermöglichen.

## Revendications

1. Transformateur (10) comprenant :
au moins un enroulement électrique (18) ;
un système de fluide comprenant un fluide (20) pour isoler l'au moins un enroulement électrique (18) ; et
un système d'analyse de gaz (12) pour déterminer les caractéristiques du gaz dissous dans le fluide (20) du système de fluide, le système d'analyse de gaz (12) comprenant un bobinage d'extraction (22) et une cellule à gaz (26) pour l'analyse du gaz, le bobinage d'extraction (22) étant agencé en contact avec le fluide (20) et comprenant un matériau perméable aux gaz pour recevoir le gaz dissous du fluide (20), le bobinage d'extraction (22) et la cellule à gaz (26) communiquant fluidiquement pour former une boucle de circulation de gaz pour faire circuler le gaz,
dans lequel la cellule à gaz (26) est agencée pour déterminer les caractéristiques du gaz extrait du fluide (20),
**caractérisé en ce que** le système d'analyse de gaz (12) comprend un module d'extraction (50) pour monter le bobinage d'extraction (22) dans un boîtier (14) du transformateur (10), le module d'extraction (50) comprenant :
un cadre de montage (52) comprenant une paroi d'engagement (56) et un bras de sonde (58) s'étendant à partir de la paroi d'engagement, dans lequel le bras de sonde (58) comprend une bobine (62) ayant le bobinage d'extraction (22) enroulé autour de la bobine (62).

2. Transformateur (10) de la revendication 1, dans lequel la boucle de circulation de gaz comprend en outre un conduit de transport (64) couplé de manière fluidique à chacun du bobinage d'extraction (22) et de la cellule à gaz (26) pour transporter le gaz reçu du fluide (20) vers la cellule à gaz (26) pour analyse.

3. Transformateur (10) de la revendication 2, comprenant en outre une source de pression motrice (54) pour faire circuler le gaz à travers le conduit de transport (64).

4. Transformateur (10) de l'une des revendications précédentes, dans lequel le bobinage d'extraction (22) est formé comme un conduit ayant un volume intérieur pour recevoir le gaz s'infiltrant à travers le matériau perméable aux gaz.

5. Transformateur (10) de la revendication 4, dans lequel une espèce de gaz qui est à la fois dans le volume intérieur et dissoute dans le fluide (20) est en équilibre.

6. Transformateur (10) de l'une des revendications précédentes, dans lequel le matériau perméable aux gaz comprend un fluoropolymère.

7. Transformateur (10) de la revendication 6, dans lequel le matériau perméable aux gaz comprend un fluoroplastique ayant au moins l'une parmi : une limite d'élasticité dans la plage allant d'environ 26 MPa à environ 29 MPa à environ 73°F, une limite d'élasticité dans la plage allant d'environ 0,5 MPa à environ 13 MPa à environ 302°F, une limite d'élasticité dans la plage allant d'environ 4 MPa à environ 13 MPa à environ 428°F, une résistance à la traction dans la plage allant d'environ 24 MPa à environ 29 MPa à environ 73°F, une résistance à la traction dans la plage allant d'environ 1 MPa à environ 15 MPa à environ 302°F, et une résistance à la traction dans la plage allant d'environ 3 MPa à environ 7 MPa à environ 428°F.

8. Transformateur (10) de l'une des revendications précédentes, dans lequel le bobinage d'extraction (22) comprend un certain nombre de boucles de bobinage permettant chacune au gaz dissous de s'y infiltrer.

9. Transformateur (10) de l'une des revendications précédentes, dans lequel la cellule à gaz (26) a une cavité destinée à recevoir le gaz pour analyse et fait partie d'un analyseur de gaz (24) qui comprend en outre une source de lumière (34) et au moins un détecteur de lumière (36, 38) destiné à recevoir la lumière provenant de la source de lumière (34).

10. Transformateur (10) de la revendication 9, dans lequel la source de lumière (34) est agencée pour faire passer la lumière d'un côté de la cellule à gaz (26) à travers le gaz à l'intérieur de la cavité (30) de la cellule à gaz (26) vers un autre côté de la cellule à gaz (26), et l'au moins un détecteur de lumière (36) est agencé sur l'autre côté pour recevoir la lumière provenant de la source de lumière (34).

11. Transformateur (10) de l'une des revendications précédentes, dans lequel :
la boucle de circulation de gaz comprend un conduit de transport (64) couplé de manière fluidique à chacun du bobinage d'extraction (22) et de la cellule à gaz (26) pour transporter le gaz entre le bobinage d'extraction (22) et la cellule à gaz (26) pour analyse,
le conduit de transport (64) comprend une pompe (54) couplée de manière fluidique au bobinage d'extraction (22) pour faire circuler le gaz à travers le conduit de transport (64), et
la pompe (54) est couplée de manière fluidique au bobinage d'extraction (22) pour fournir une pression inférieure au niveau d'une sortie du bobinage d'extraction (22) par rapport à une pression à l'intérieur de la cellule à gaz (26).

12. Transformateur (10) de la revendication 11, dans lequel :
la pompe (54) est montée sur le cadre de montage (52).

13. Transformateur (10) de la revendication 12, dans lequel :
le bobinage d'extraction (22) est enroulé autour de la bobine (62) pour former un certain nombre de tours de bobinage ayant un agencement empilé successivement pour l'exposition au fluide (20).

14. Transformateur (10) de la revendication 13, dans lequel :
la bobine (62) comprend un lit de bobine annulaire (61) pour recevoir le bobinage d'extraction (22) enroulé sur celle-ci, dans lequel le lit de bobine annulaire (61) définit des ouvertures (63) s'étendant à travers le lit de bobine annulaire (61) qui permet au fluide (20) d'entrer en contact avec les parties intérieures du bobinage d'extraction (22).

15. Transformateur (10) de la revendication 14, dans lequel :
la bobine (62) comprend une entretoise (65) traversant radialement le lit de bobine annulaire (61) pour fournir un support structurel et définissant des ouvertures (67) pour permettre la circulation du fluide (20) à travers la bobine (62).
